# FASCICULE DE BREVET EUROPEEN

(11) **EP 0 433 131 B1**
(45) Date de publication et mention de la délivrance du brevet: **20.10.1993**
(21) Numéro de dépôt: 90403446.9
(22) Date de dépôt: 04.12.1990
(51) Int. Cl.: A61K 7/00, A61K 9/127

(54) **Composition cosmétique pour le soin des cheveux et utilisation de ladite composition**
Kosmetisches Mittel zur Haarpflege und Verwendung dieses Mittels
Cosmetic composition for haircare and use of said composition

(30) Priorité: 13.12.1989 FR 8916481
(43) Date de publication de la demande: 19.06.1991
(73) Titulaire: L'OREAL, 75008 Paris (FR)
(72) Inventeur: Grollier, Jean-François, F-75004 Paris (FR); Richoux, Isabelle, F-75017 Paris (FR)
(74) Mandataire: Peuscet, Jacques

(56) Documents cités:
- EP-A- 0 347 306
- FR-A- 2 485 921

## Description

La présente invention concerne une composition cosmétique pour le soin des cheveux et l'utilisation de ladite composition comme produit à rincer ou comme produit non rincé.

On sait que certains lipides amphiphiles sont susceptibles de former, en présence d'une phase aqueuse, une phase lamellaire, puis, par agitation, des vésicules en dispersion dans ladite phase aqueuse. Les vésicules obtenues sont formées d'un ou, le plus souvent, de plusieurs feuillets concentriques (couches bimoléculaires ou multimoléculaires) de lipides encapsulant une phase aqueuse. Les lipides amphiphiles utilisés peuvent être ioniques ou non-ioniques. De telles dispersions de vésicules, ainsi que certains de leurs modes de préparation sont, par exemple, décrits dans le brevet français 2 315 991. On a proposé d'utiliser ces dispersions aqueuses de vésicules de lipides amphiphiles pour le traitement des cheveux, mais ces compositions ne permettent pas de donner aux cheveux une douceur satisfaisante.

Il est également connu, par les brevets français 2 485 921 et 2 490 504, que les vésicules de lipides amphiphiles stabilisent en phase aqueuse des dispersions de lipides non-miscibles à l'eau, en particulier d'huiles végétales, sans qu'il soit nécessaire d'ajouter un agent émulsifiant. Mais les compositions cosmétiques obtenues avec les huiles végétales pour le soin des cheveux ont tendance à amollir et alourdir les cheveux et il est impossible, par la suite, d'obtenir une coiffure ayant de la tenue et du volume.

Il est, par ailleurs, décrit dans EP-A 347 306, qui fait partie de l'état de la technique selon l'article 54.3 de la CBE, un parfum à base de vésicules de lipides amphiphiles ioniques et d'huiles essentielles comme parfum, composition dans laquelle le rapport phase lipidique/parfum est compris entre 0,05 et 0,2.

La présente invention concerne une composition permettant de remédier aux inconvénients précités.

La présente invention a pour objet une composition cosmétique pour le soin des cheveux, caractérisée par le fait qu'elle contient dans une phase aqueuse continue de dispersion renfermant éventuellement au moins un additif cosmétiquement acceptable:
a) des vésicules préparées à partir d'une phase lipidique comprenant au moins un lipide amphiphile, ionique ou non-ionique, associé éventuellement à au moins un additif stabilisant, lesdites vésicules contenant une phase aqueuse encapsulée; et
b) au moins une huile essentielle, naturelle ou synthétique, sous forme de gouttelettes dispersées dans la phase aqueuse de dispersion, ladite (ou lesdites) huile(s) essentielle(s) étant présente(s) à raison de 0,2 à 20 % en poids par rapport au poids total de la composition et le rapport pondéral de la phase lipidique à l'(ou aux) huile(s) essentielle(s) étant compris entre 0,3 et 10 et de préférence entre O,3 et 5.

Selon la présente invention, on a trouvé que les compositions selon l'invention contenant des vésicules de lipides amphiphiles et au moins une huile essentielle confèrent aux cheveux plus de douceur et de brillance que les compositions ne contenant que des vésicules de lipides amphiphiles et que l'application de ces compositions sur des cheveux propres, mouillés ou séchés, apporte un effet traitant à la chevelure sans nuire ni à la légèreté, ni à la brillance d'une coiffure naturelle, ce qui n'est pas le cas avec les compositions antérieurement décrites renfermant une association de vésicules et d'une huile végétale, qui chargent les cheveux et alourdissent la chevelure.

Les lipides amphiphiles susceptibles de former des vésicules sont des lipides ioniques ou non-ioniques. Parmi les lipides ioniques utilisables, on peut citer les phospholipides naturels, tels que lécithine d'oeuf, lécithine de soja ou sphingomyéline, et les phospholipides de synthèse, tel que dipalmitoyl-phosphatidylcholine ou lécithine hydrogénée.

Le lipide amphiphile est, de préférence, un lipide non-ionique et, mieux encore, il est constitué par un dérivé de polyglycérol, linéaire ou ramifié, de formule (I) : 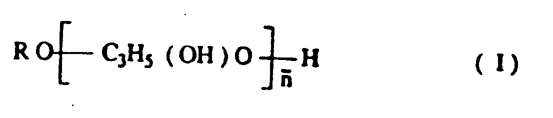
formule dans laquelle :
**-C₃H₅(OH)O-** représente les structures suivantes prises en mélange ou séparément :

**-CH₂-CHOH-CH₂O-**

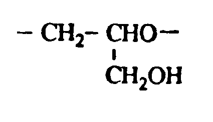

et 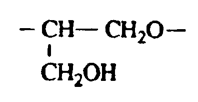
- n̅ est une valeur statistique moyenne comprise entre 2 et 6 et
- R est :
   a) soit une chaîne aliphatique R₁ ou un reste R₂CO, R₁ étant un radical aliphatique, linéaire ou ramifié, en C₁₂-C₁₈ et R₂ étant un radical aliphatique, linéaire ou ramifié, en C₁₁-C₁₇ ;
   b) soit

      **R₃⁅O-C₂H₃(R₄)⁆**

où **-O-C₂H₃(R₄)-** représente les structures suivantes prises en mélange ou séparément : 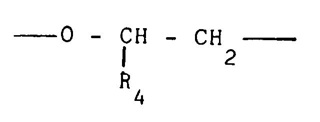
et 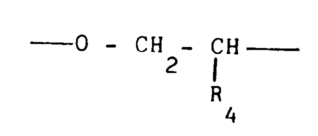
- R₃ étant un radical R₁ ou R₂CO ;
- R₄ étant un radical R₁ ; et
- R₁ et R₂ ayant les significations données ci-dessus.

On peut, selon l'invention, utiliser un mélange de lipides ionique(s) et non-ionique(s).

On peut également, à côté des lipides amphiphiles ioniques ou/et non-ioniques décrits plus haut, ajouter des mélanges constitués, en particulier, de sphingomyélines, de cérébrosides, de stérols, de phosphatidylcholine, de phosphatidylsérine, de phosphatidyléthanolamine et de glycolipides.

L'additif stabilisant est destiné, de façon connue, à modifier la perméabilité et/ou la charge superficielle des vésicules. Il est, de préférence, choisi dans le groupe formé par les stérols et les stabilisants anioniques. Le stérol est avantageusement le cholestérol. Le stabilisant anionique est avantageusement choisi d'une part, parmi les sels monosodiques ou disodiques des acylglutamates, le radical acyle étant en C₁₄-C₂₂, tels que le sel monosodique du stéaroylglutamate et les sels disodiques avec des radicaux acyles du coprah et du suif ou encore les radicaux stéaroyle et cocoyle, et d'autre part, parmi les esters phosphoriques d'alcools gras en C₁₂-C₂₂. De façon connue, on peut ajouter au(x) lipide (s) amphiphile(s) à la fois un stérol et un stabilisant anionique.

Les vésicules ont avantageusement une dimension moyenne comprise entre 10 et 1000 nm.

La phase lipidique représente, de préférence, de 0,1 à 8 %, et mieux encore, de 0,25 à 3 % du poids total de la composition; le(s) lipide(s) amphiphile(s) ionique(s) et/ou non-ionique(s) représente(nt) de préférence 0,1 à 6 % et mieux encore de O,1 à 2,5 % du poids total de la composition; et l'additif stabilisant représente avantageusement moins de 8 % du poids total de la composition et, de préférence, de O,1 à 8 % de ce poids.

Les huiles essentielles naturelles sont des produits obtenus à partir de matières premières d'origine végétale (feuilles, tiges, fleurs, rameaux de plantes et/ou plantes entières), soit par entraînement à la vapeur d'eau, sèche ou humide, soit par des procédés mécaniques, soit par distillation à sec, soit par extraction au moyen de solvant volatil (voir la norme NF-T-75006). Les huiles essentielles se distinguent des huiles végétales par le fait qu'elles ne peuvent pas être décomposées par saponification en glycérol et savon d'acide gras.

Selon la présente invention, l'huile essentielle peut être avantageusement choisie parmi les huiles d'eucalyptus, de lavandin, de lavande, de vétiver, de litsea cubeba, de citron, de santal, de thym rouge ou blanc, de romarin, de camomille, de sarriette, de noix de muscade, de cannelle, d'hysope, de carvi, d'orange, de girofle, de menthe, de rose et de graines de persil.

Les gouttelettes d'huile(s) essentielle(s) ont avantageusement une dimension moyenne comprise entre 100 et 10000 nm.

Les huiles essentielles ont pour fonction principale d'améliorer la douceur, l'aspect et le coiffant des cheveux. Cependant, certaines des huiles essentielles ont, en plus, une activité antipelliculaire, antichute des cheveux et/ou antiparasitaire, notamment antipoux; d'autres huiles, prises isolément ou en mélange, ont une action relaxante ou stimulante.

Selon la présente demande, la phase aqueuse encapsulée et/ou la phase lipidique des vésicules peuvent contenir des actifs cosmétiques et/ou pharmaceutiques qui peuvent, notamment, avoir pour effet d'augmenter encore l'activité connue de l'huile essentielle. Ces agents sont, par exemple, des agents antichute des cheveux, parmi lesquels on peut citer le nicotinate de méthyle, ou des agents antipelliculaires, tels que le sel d'éthanolamine de l'hydroxy-1 méthyl-4 (triméthyl-2,4,4)-pentyl-6 1 H-pyridinone-2.

La phase de dispersion de la composition selon l'invention peut contenir au moins un agent épaississant pour faciliter l'application et la localisation des compositions sur les cheveux. Parmi les agents épaississants utilisables, on peut citer les dérivés cellulosiques, la gomme de xanthane, et, plus particulièrement, les acides polyacryliques réticulés tels que ceux vendus sous la dénomination commerciale "CARBOPOL®" (par exemple "CARBOPOL® 934" et "CARBOPOL® 940") par la société "GOODRICH".

La phase aqueuse de dispersion peut également contenir selon l'invention des additifs cosmétiquement acceptables bien connus tels que conservateurs, parfums, agents acidifiants, agents alcalinisants, stabilisants, colorants et filtres solaires.

Les compositions selon l'invention peuvent être utilisées sous forme de produits à rincer à appliquer après un shampooing, après une coloration ou une décoloration, après une permanente ou un défrisage; dans ce cas, la teneur en huile(s) essentielle(s) de la composition est, de préférence, comprise entre 3 à 2O % en poids par rapport au poids total de la composition. Les compositions selon l'invention peuvent aussi être utilisées sous forme de produits non rincés, par exemple avant une mise en plis, un brushing ou en finition de coiffage; dans ce cas, la teneur en huile(s) essentielle(s) de la composition est, de préférence, comprise entre O,2 et 3 % en poids par rapport au poids total de la composition.

Les compositions selon l'invention se présentent sous forme de liquides plus ou moins épaissis, de gels, de crèmes ou d'aérosols.

Les exemples donnés ci-dessous, à titre purement illustratif, permettront de mieux comprendre l'invention.

### EXEMPLE 1 (comparatif)

### I) Préparation des compositions testées :

### A) Préparation d'une composition A selon l'invention.

Dans une première étape, on fond, en agitant doucement à une température de 80-90°C, un mélange de 0,375 g de lipides non-ioniques de formule: 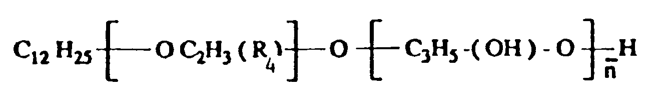
formule dans laquelle :
**―C₃H₅(OH)O-** est constitué par un mélange de radicaux : 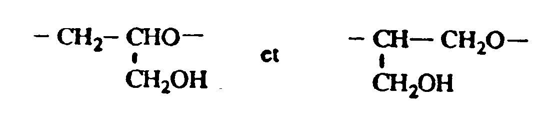
**-O-C₂H₃(R₄)-** est constitué par un mélange de radicaux : 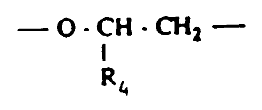
et 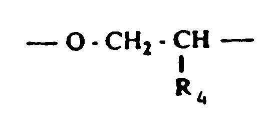
- n̅ = 6 ;
- R₄ est un mélange de radicaux C₁₄H₂₉ et C₁₆H₃₃ avec 0,1 g de "SPHINGOCERYL"®, qui est un mélange commercialisé sous ce nom par "les Laboratoires Sérobiologiques de Nancy", qui contient des sphingomyélines, des cérébrosides, des stérols, la phosphatidylcholine, la phosphatidylsérine, la phosphatidyléthanolamine et des glycolipides.

On introduit dans le mélange fondu 0,95 g d'eau portée à 90°C, contenant un conservateur, et l'on mélange pendant environ 6 à 8 minutes au moyen d'un agitateur "ULTRA TURRAX®. A la phase ainsi obtenue, on ajoute 1,43 g d'eau à température ambiante, puis on homogénéise le mélange à l'"ULTRA TURRAX® pendant encore environ 8 minutes avant de laisser revenir le mélange à la température ambiante. On obtient ainsi une dispersion de vésicules dans une phase aqueuse.

Dans une seconde étape, on additionne à la phase aqueuse, goutte à goutte, 0,375 g d'huile essentielle d'eucalyptus en agitant à l'"ULTRA TURRAX®. Puis on ajoute un gel aqueux bien homogène constitué de 0,25 g d'agent épaississant vendu sous la dénomination commerciale "CARBOPOL 934"® par la société "GOODRICH", dissous dans 5 g d'eau renfermant des conservateurs et ajusté à pH 7 avec de l'amino-2 méthyl-2 propanol-1 et on complète à 100 g avec de l'eau.

### B) Préparation d'une composition B non conforme à l'invention.

On a préparé une composition B identique à celle décrite en A, mais sans ajouter d'huile essentielle dans la seconde étape.

### C) Préparation d'une composition C non conforme à l'invention.

On a également préparé une composition C identique à celle décrite en A, sauf que l'huile essentielle d'eucalyptus a été remplacée par de l'huile de colza.

### II) ESSAIS COMPARATIFS

On a d'abord effectué des tests sur une mèche de 5 g en appliquant, sur des mèches mouillées et essorées, 0,5 g de composition A et sur d'autres mèches mouillées 0,5 g de composition B et en séchant ensuite pendant 15 minutes à 60°C. On a ensuite effectué une évaluation tactile avec un panel de neuf testeurs. Neuf testeurs sur neuf trouvent les mèches traitées par la composition A plus douce.

On a également effectué des essais comparatifs d'application des compositions A et C. Les essais ont été effectués sur tête en appliquant 2 g de composition A sur les cheveux secs d'une demi-tête et 2 g de composition B sur les cheveux secs de l'autre demi-tête d'un modèle.

Les neuf testeurs ont déclaré à l'unanimité, pour les 10 modèles testés qu'ils préféraient les cheveux traités par la composition A pour leur légéreté, leur brillance et pour le naturel de la coiffure.

### EXEMPLE 2

Dans une première étape, on fond, en agitant doucement à une température de 80-90°C, un mélange de 0,705 g de lipides non-ioniques de formule : 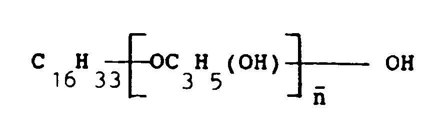
où -OC₃H₅(OH) est constitué par un mélange des radicaux 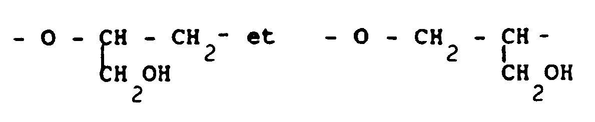
et n̅ est une valeur statistique moyenne égale à 3,
avec 0,705 g de cholestérol et 0,09 g du sel monosodique du glutamate de formule : 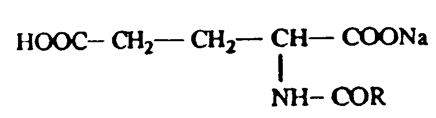
dans laquelle R est un mélange de radicaux alkényle et/ou alkyle en C₁₃ - C₂₁ dérivé des acides gras du suif, vendu sous la dénomination commerciale "ACYLGLUTAMATE HS 11"® par la société "AJINOMOTO".

On introduit dans le mélange fondu 3 g d'eau portée à 90°C, contenant un conservateur, et l'on mélange pendant environ 6 à 8 minutes au moyen d'un agitateur "ULTRA TURRAX"®. A la phase ainsi obtenue, on ajoute 4,5 g d'eau à température ambiante, puis on homogénéise le mélange à l'"ULTRA TURRAX"® pendant encore environ 8 minutes avant de laisser revenir le mélange à la température ambiante. On obtient ainsi une dispersion de vésicules dans une phase aqueuse.

Dans une seconde étape, on additionne goutte à goutte 1,5 g d'un mélange d'huiles essentielles (citron : plus de 20 % ; lavande et santal : de 5 à 20 % ; thym blanc : jusqu'à 1 %) (proportions en poids) vendu sous la dénomination commerciale "COCKTAIL B (HGB 18)"® par la société "CHARABOT", en agitant à l'"ULTRA TURRAX"®. Puis on ajoute un gel aqueux bien homogène constitué de 0,5 g d'un agent épaississant vendu sous la dénomination commerciale "CARBOPOL 934"® par la société "GOODRICH", dissous dans 10 g d'eau renfermant des conservateurs et des colorants ; on ajuste le pH à 7 avec de l'amino-2 méthyl-2 propanol-1 et on complète à 100 g avec de l'eau.

On applique cette composition à raison d'environ 4 à 5 g par tête sur des cheveux lavés et essorés. Cette composition de soin a une action antipelliculaire et permet d'obtenir des cheveux doux au toucher, légers et brillants, qui ont un coiffant naturel.

### EXEMPLE 3

Dans une première étape, on fond, en agitant doucement à une température de 80 - 90°C, un mélange de 1,06 g de lipides amphiphiles non-ioniques de formule : 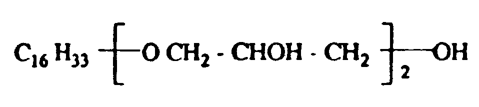
avec 1,06 g de cholestérol et 0,14 g du sel monosodique du glutamate de formule : 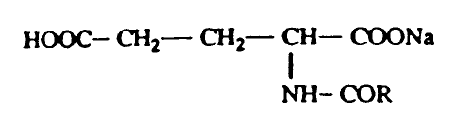
dans laquelle R est un mélange de radicaux alkényle et/ou alkyle hydrogénés en C₁₃- C₂₁ dérivé des acides gras du suif, vendu sous la dénomination commerciale "ACYLGLUTAMATE HS 11"® par la société "AJINOMOTO", et 0,6 g de **"SPHINGOCERYL".**®

On introduit ensuite dans le mélange fondu 5,72 g d'eau portée à 90°C contenant un conservateur et l'on mélange pendant environ 6 à 8 minutes au moyen d'un agitateur "ULTRA TURRAX"®. A la phase ainsi obtenue, on ajoute 8,58 g d'eau à température ambiante puis on homogénéise le mélange à l'"ULTRA TURRAX"® pendant encore environ 8 minutes avant de laisser revenir le mélange à la température ambiante. On obtient ainsi une dispersion de vésicules dans une phase aqueuse.

Dans une seconde étape, on additionne goutte à goutte 2,25 g d'huiles essentielles (citron : plus de 20 % ; lavande : plus de 20 % ; hysope : 1 à 5 % ; thym : de 1 à 5 % ; noix de muscade : 1 à 5 %) (proportions pondérales), ce mélange étant vendu sous la dénomination commerciale "COCKTAIL A (OR 12504)"® par la société "MANE", en agitant à l'"ULTRA TURRAX"®. Puis on ajoute un gel aqueux bien homogène constitué de 0,5 g d'agent épaississant vendu sous la dénomination commerciale "CARBOPOL 934"® par la société "GOODRICH", dissous dans 10 g d'eau renfermant des conservateurs et des colorants ; on ajuste le pH à 7 avec de l'amino-2 méthyl-2 propanol-1 et on complète à 100 g avec de l'eau.

Cette composition qui a, par ailleurs, une action antichute des cheveux apporte les mêmes effets cosmétiques que la composition de l'exemple 2.

### EXEMPLE 4 :

Dans une première étape, on fond, en agitant doucement à une température de 80 - 90°C, un mélange de 0,225 g de lipides amphiphiles non-ioniques de formule : 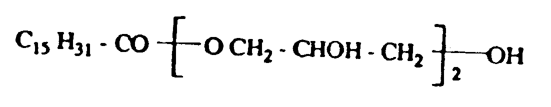
avec 0,225 g de cholestérol et 0,025 g du sel monosodique du glutamate de formule : 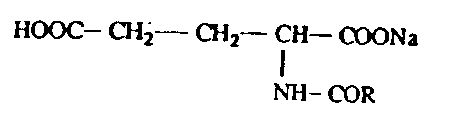
formule dans laquelle R est un mélange de radicaux alkényle et/ou alkyle en C₁₃- C₂₁ dérivé des acides gras du suif, vendu sous la dénomination commerciale "ACYLGLUTAMATE HS 11"® par la société "AJINOMOTO".

On introduit dans le mélange fondu 0,95 g d'eau portée à 90°C contenant un conservateur et l'on mélange pendant environ 6 à 8 minutes au moyen d'un agitateur "ULTRA TURRAX"®. A la phase ainsi obtenue, on ajoute 1,43 g d'eau à température ambiante, puis on homogénéise le mélange à l'"ULTRA TURRAX"® pendant encore environ 8 minutes avant de laisser revenir le mélange à la température ambiante. On obtient ainsi une dispersion de vésicules dans une phase aqueuse.

Dans une seconde étape, on additionne goutte à goutte 0,375 g d'huile essentielle d'eucalyptus en agitant à l'"ULTRA TURRAX"®. Puis on ajoute un gel aqueux bien homogène constitué de 0,25 g d'agent épaississant vendu sous la dénomination commerciale "CARBOPOL 934"® par la société "GOODRICH", dissous dans 5 g d'eau renfermant des conservateurs ; on ajuste le pH à 7 avec de l'amino-2 méthyl-2 propanol-1 et on complète à l'eau à 100 g.

Cette composition de soin apporte les mêmes effets cosmétiques que la composition de l'exemple 2, c'est-à-dire que les cheveux sont doux, légers et brillants et ont un aspect naturel.

### EXEMPLE 5

Dans une première étape, on fond en agitant doucement à une température de 60°C, 0,3 g de lécithine de soja à 75 % de phosphatidylcholine vendue par la Société SEPPIC sous la dénomination commerciale "LIPOID S75"®.

On introduit dans le mélange fondu 0,96 g d'eau portée à 80°C contenant un conservateur et l'on mélange pendant environ 5 mn à l'aide d'un agitateur "ULTRA TURRAX"®, on laisse gonfler le mélange pendant une heure. A la phase ainsi obtenue, on ajoute 1,44 g d'eau à 20°C ; on agite le mélange pendant quelques minutes à l'aide du même agitateur. On complète par 0,7 g d'eau à 20°C sous agitation à l'"ULTRA TURRAX"®.

On obtient ainsi une dispersion de vésicules dans une phase aqueuse.

Dans une seconde étape on additionne goutte à goutte en agitant à l'agitateur "ULTRA TURRAX"®, 0,4 g d'un mélange d'huiles essentielles vendu par la Société MANE sous la dénomination commerciale "COCKTAIL C (OR 12509)"®, ce mélange d'huiles essentielles est défini comme suit (en poids) :
- Citron : plus de 20 %
- Girofle : 1 à 5 %
- Menthe : 1 à 5 %
- Rose : 1 à 5 %
- Lavande : 5 à 20 %

Puis on ajoute un gel aqueux bien homogène constitué de 0,5 g d'agent épaississant vendu sous la dénomination commerciale "CARBOPOL 934"® par la Société GOODRICH dissous dans 10 g d'alcool à 96° renfermant des conservateurs et des colorants ; on ajuste le pH à 7 avec 2-amino 2-méthyl 1-propanol et on complète à 100 g avec de l'eau.

On applique cette composition à raison d'environ 4 à 5 g par tête sur des cheveux lavés et essorés. Cette composition qui a, par ailleurs, une action stimulante, apporte les mêmes effets cosmétiques que la composition de l'exemple 2.

### EXEMPLE 6 :

Dans une première étape, on fond, en agitant doucement à une température de 85°C, 0,4 g de phytostérol polyoxyéthyléné à 5 moles d'oxyde d'éthylène vendu par la Société HENKEL sous la dénomination commerciale "GENEROL 122 E 5"®, puis au mélange fondu, on additionne 0,6 g de lécithine hydrogénée à 30-35 % de phosphatidylcholine hydrogénée vendue par la Société NIKKO sous la dénomination commerciale "LECINOL S 10"® et ceci jusqu'à homogénéisation parfaite (5 mn).

On introduit dans le mélange fondu, 3 g d'eau portée à 80°C contenant un conservateur et l'on mélange pendant environ 5 mn au moyen d'un agitateur "ULTRA TURRAX"®, puis on laisse gonfler le mélange pendant une heure. A la phase ainsi obtenue, on ajoute 4,5 g d'eau à 20°C ; on agite le mélange pendant encore quelques minutes au moyen du même agitateur. On obtient une dispersion de vésicules dans une phase aqueuse.

Dans une seconde étape, on additionne goutte à goutte, en agitant à l'agitateur "ULTRA TURRAX"®, 0,5 g d'un mélange d'huiles essentielles vendu par la Société ROBERTET sous la dénomination commerciale "COCKTAIL F (8388)"®; ce mélange d'huiles essentielles est défini comme suit (en poids) :
- Lavande : plus de 75 %
- Camomille du Maroc : 10 à 20 %
- Orange de Floride : 10 à 20 %
- Graines de Persil : 1 à 5 %
- Rose : moins de 1 %

Puis on ajoute un gel aqueux bien homogène constitué de 0,5 g d'agent épaississant vendu sous la dénomination commerciale "CARBOPOL 934"® par la Société GOODRICH dissous dans 10 g d'alcool à 96° renfermant des conservateurs et des colorants ; on ajuste le pH à 7 avec 2-amino 2-méthyl l-propanol et on complète à 100 g avec de l'eau.

On applique cette composition à raison d'environ 4 à 5 g par tête sur des cheveux lavés et essorés. Cette composition qui a, par ailleurs, une action relaxante apporte les mêmes effets cosmétiques que la composition de l'exemple 2.

## Revendications

1. Composition cosmétique pour le soin des cheveux caractérisée par le fait qu'elle contient, dans une phase aqueuse continue de dispersion :
a) des vésicules préparées à partir d'une phase lipidique comprenant au moins un lipide amphiphile, ionique ou non-ionique, associé éventuellement à au moins un additif stabilisant, lesdites vésicules contenant une phase aqueuse encapsulée; et
b) au moins une huile essentielle, naturelle ou synthétique, sous forme de gouttelettes dispersées dans la phase aqueuse de dispersion, ladite (ou lesdites) huile(s) essentielle(s) étant présente(s) à raison de 0,2 à 20 % en poids par rapport au poids total de la composition et le rapport pondéral de la phase lipidique à l'(ou aux) huile(s) essentielle(s) étant compris entre 0,3 et 10.

2. Composition selon la revendication 1, caractérisée par le fait que le rapport pondéral de la phase lipidique à l'huile essentielle est compris entre 0,3 et 5.

3. Composition selon l'une des revendications 1 ou 2, caractérisée par le fait que le lipide amphiphile est un lipide amphiphile non-ionique constitué par un dérivé de polyglycérol, linéaire ou ramifié, de formule (I) : 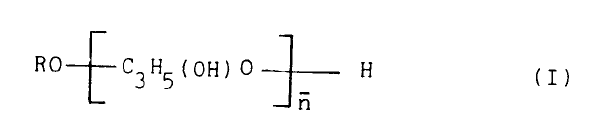 formule dans laquelle :
-C₃H₅(OH)O- représente les structures suivantes prises en mélange ou séparément :
**-CH₂-CHOH-CH₂O-** 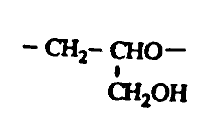
et 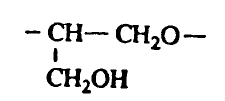
- n̅ est une valeur statistique moyenne comprise entre 2 et 6 et
- R est :
a)soit une chaîne aliphatique R₁ ou un reste R₂ CO, R₁ étant un radical aliphatique, linéaire ou ramifié, en C₁₂-C₁₈ et R₂ étant un radical aliphatique, linéaire ou ramifié, en C₁₁-C₁₇ ;
b) soit
**R₃⁅O-C₂H₃(R₄)⁆**
où **-O-C₂H₃(R₄)-** représente les structures suivantes prises en mélange ou séparément : 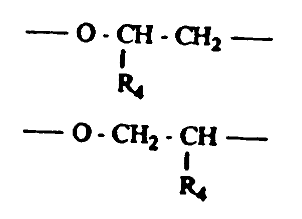 R₃ étant un radical R₁ ou R₂CO ;
R₄ étant un radical R₁ ; et
R₁ et R₂ ayant les significations données ci-dessus.

4. Composition selon l'une des revendications 1 à 3, caractérisée par le fait qu'elle contient des vésicules préparées à partir d'un mélange de lipides amphiphiles ioniques et non-ioniques.

5. Composition selon l'une des revendications 1 à 4, caractérisée par le fait que l'additif stabilisant est choisi dans le groupe formé par les stérols et les stabilisants anioniques.

6. Composition selon la revendication 5, caractérisée par le fait que le stérol est le cholestérol.

7. Composition selon la revendication 5, caractérisée par le fait que le stabilisant anionique est choisi parmi les sels monosodiques ou disodiques de glutamate d'acyle, le radical acyle étant en C₁₄-C₂₂ et les esters phosphoriques d'alcools gras en C₁₂-C₂₂.

8. Composition selon l'une des revendications 1 à 7, caractérisée par le fait que la phase lipidique représente de 0,1 à 8 % en poids du poids total de la composition.

9. Composition selon l'une des revendications 1 à 8, caractérisée par le fait que les lipides amphiphiles représentent de 0,1 à 6 % en poids du poids total de la composition.

10. Composition selon l'une des revendications 1 à 9, caractérisée par le fait que l'additif stabilisant représente moins de 8 % en poids du poids total de la composition.

11. Composition selon l'une des revendications 1 à 10, caractérisée par le fait que l'huile essentielle est choisie dans le groupe formé par les huiles d'eucalyptus, de lavandin, de lavande, de vétiver, de litsea cubeba, de citron, de santal, de thym rouge ou blanc, de romarin, de camomille, de sarriette, de noix de muscade, de cannelle, d'hysope, de carvi, d'orange, de girofle, de menthe, de rose et de graines de persil.

12. Composition selon l'une des revendications 1 à 11, caractérisée par le fait que la phase aqueuse encapsulée et/ou la phase lipidique de vésicules contiennent des actifs cosmétiques et/ou pharmaceutiques.

13. Composition selon l'une des revendications 1 à 12, caractérisée par le fait que la phase aqueuse de dispersion contient au moins un additif choisi dans le groupe formé par les agents épaississants, les conservateurs, les parfums, les agents acidifiants, les agents alcalinisants, les stabilisants, les colorants et les filtres solaires.

14. Composition selon l'une des revendications 1 à 13, caractérisée par le fait que les vésicules ont une dimension moyenne comprise entre 10 et 1000 nm et que les gouttelettes d'huile(s) essentielle(s) ont une dimension moyenne comprise entre 100 et 10000 nm.

15. Utilisation d'une composition selon l'une des revendications 1 à 14, sous forme de produit à rincer, la teneur de la composition en huile(s) essentielle(s) étant comprise entre 3 et 20% en poids par rapport au poids total de la composition.

16. Utilisation d'une composition selon l'une des revendications 1 à 14, sous forme de produit non rincé, la teneur de la composition en huile(s) essentielle(s) étant comprise entre 0,2 et 3% en poids par rapport au poids total de la composition.

## Claims

1. Cosmetic composition for hair care, characterised in that it contains, in a continuous aqueous dispersion phase:
a) vesicles prepared from a lipid phase comprising at least one amphiphilic, ionic or nonionic lipid, optionally combined with at least one stabilising additive, the said vesicles containing an encapsulated aqueous phase; and
b) at least one natural or synthetic essential oil in the form of droplets dispersed in the aqueous dispersion phase, the said essential oil(s) being present at a concentration of 0.2 to 20 % by weight with respect to the total weight of the composition and the ratio by weight of the lipid phase to the essential oil(s) being between 0.3 and 10.

2. Composition according to Claim 1, characterised in that the ratio by weight of the lipid phase to the essential oil is between 0.3 and 5.

3. Composition according to one of Claims 1 or 2, characterised in that the amphiphilic liquid is a nonionic amphiphilic lipid consisting of a linear or branched polyglycerol derivative of formula (I): 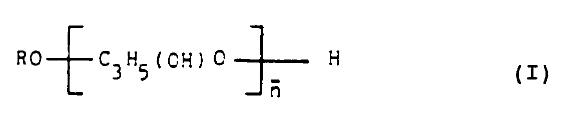 in which formula:
- C₃H₅(OH)O - represents the following structures taken as a mixture or separately:
**-CH₂-CHOH-CH₂O-** 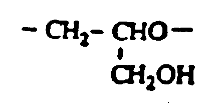
and 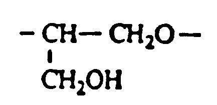
- n̅ is a mean statistical value between 2 and 6 and
- R is:
a) either an aliphatic chain R₁ or a residue R₂CO, R₁ being a linear or branched, C₁₂-C₁₈ aliphatic radical; and R₂ being a linear or branched, C₁₁-C₁₇ aliphatic radical;
b) or
**R₃⁅O-C₂H₃(R₄)⁆**
where **-O-C₂H₃(R₄)-** represents the following structures taken as a mixture or separately: 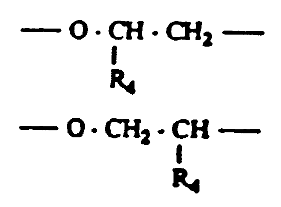 R₃ being a radical R₁ or R₂CO;
R₄ being a radical R₁; and
R₁ and R₂ having the meanings given above.

4. Composition according to one of Claims 1 to 3, characterised in that it contains vesicles prepared from a mixture of ionic and nonionic amphiphilic lipids.

5. Composition according to one of Claims 1 to 4, characterised in that the stabilising additive is chosen from the group formed by sterols and anionic stabilising agents.

6. Composition according to Claim 5, characterised in that the sterol is cholesterol.

7. Composition according to Claim 5, characterised in that the anionic stabilising agent is chosen from monosodium or disodium salts of acylglutamate, the acyl radical being C₁₄-C₂₂, and phosphoric esters of C₁₂-C₂₂ fatty alcohols.

8. Composition according to one of Claims 1 to 7, characterised in that the lipid phase represents from 0.1 to 8 % by weight of the total weight of the composition.

9. Composition according to one of Claims 1 to 8, characterised in that the amphiphilic lipids represent from 0.1 to 6 % by weight of the total weight of the composition .

10. Composition according to one of Claims 1 to 9, characterised in that the stabilising additive represents less than 8 % by weight of the total weight of the composition.

11. Composition according to one of Claims 1 to 10, characterised in that the essential oil is chosen from the group formed by the oils of eucalyptus, lavandin, lavender, vetiver, litsea cubeba, lemon, sandalwood, red or white thyme, rosemary, camomile, savory, nutmeg, cinnamon, hyssop, caraway, orange, cloves, mint, rose and parsley seeds.

12. Composition according to one of Claims 1 to 11, characterised in that the encapsulated aqueous phase and/or the lipid phase of vesicles contain cosmetic and/or pharmaceutical active ingredients.

13. Composition according to one of Claims 1 to 12, characterised in that the aqueous dispersion phase contains at least one additive chosen from the group formed by thickening agents, preserving agents, fragrances or flavours, acidifying agents, basifying agents, stabilising agents, dyes and sunscreening agents.

14. Composition according to one of Claims 1 to 13, characterised in that the vesicles have a mean size between 10 and 1000 nm and in that the droplets of essential oil(s) have a mean size between 100 and 10,000 nm.

15. Use of a composition according to one of Claims 1 to 14, in the form of a rinsing product, the content of the composition in essential oil(s) being between 3 and 20 % by weight with respect to the total weight of the composition.

16. Use of a composition according to one of Claims 1 to 14, in the form of a nonrinsed product, the content of the composition in essential oil(s) being between 0.2 and 3 % by weight with respect to the total weight of the composition.

## Patentansprüche

1. Kosmetisches Mittel zur Haarpflege, dadurch gekennzeichnet, daß es in einer kontinuierlichen wäßrigen Dispersionsphase enthält:
a) Vesikel, hergestellt aus einer Lipidphase, umfassend wenigstens ein amphiphiles, ionisches oder nicht-ionisches Lipid, gegebenenfalls in Kombination mit wenigstens einem stabilisierenden Zusatz, wobei die Vesikel eine eingeschlossene wäßrige Phase enthalten; und
b) wenigstens ein ätherisches, natürliches oder synthetisches Öl, in Form von in der wäßrigen Dispersionsphase dispergierten Tröpfchen, wobei das (oder die) ätherische(n) Öl(e) in einem Anteil von 0,2 bis 20 Gew.-% bezogen auf das Gesamtgewicht des Mittels enthalten ist (sind) und wobei das Gewichtsverhältnis von der Lipidphase zu dem (den) ätherischen Öl(en) im Bereich von 0,3 bis 10 liegt.

2. Mittel nach Anspruch 1, dadurch gekennzeichnet, daß das Gewichtsverhältnis von Lipidphase zu ätherischem Öl im Bereich von 0,3 bis 5 liegt.

3. Mittel nach einem der Ansprüche 1 oder 2, dadurch gekennzeichnet, daß das amphiphile Lipid ein nicht-ionisches amphiphiles Lipid ist, welches von einem geradkettigen oder verzweigten Polyglycerinderivat der Formel (I) 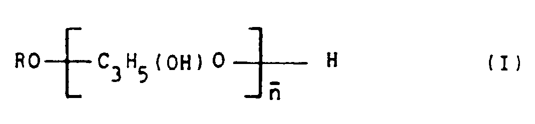 gebildet wird, worin - C₃H₅(OH)O - die folgenden Strukturen einzeln oder im Gemisch aufweist:
-CH₂-CHOH-CH₂O- 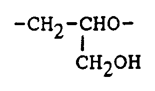
und 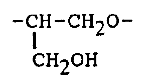 worin n̅ für einen statistischen Mittelwert von 2 bis 6 steht und
R für
a) eine aliphatische Kette R₁ oder einen Rest R₂CO steht, worin R₁ einen geradkettigen oder verzweigten C₁₂ - C₁₈-Rest bedeutet und R₂ für einen geradkettigen oder verzweigten C₁₁ - C₁₇-Rest steht;
b) oder R₃ ⁅O-C₂H₃(R₄)⁆ steht,
worin -O-C₂H₃(R₄)- für folgende Strukturen einzeln oder im Gemisch steht 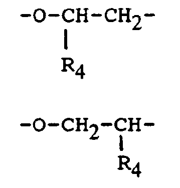 worin R₃ für einen Rest R₁ oder R₂CO steht;
R₄ für einen Rest R₁ steht; und
R₁ und R₂ die oben genannten Bedeutungen besitzen.

4. Mittel nach einem der Ansprüche 1 bis 3, dadurch gekennzeichnet, daß es Vesikel enthält, die aus einem Gemisch von ionischen und nicht-ionischen amphiphilen Lipiden hergestellt werden.

5. Mittel nach einem der Ansprüche 1 bis 4, dadurch gekennzeichnet, daß der stabilisierende Zusatz ausgewählt ist unter Sterolen und anionischen Stabilisatoren.

6. Mittel nach Anspruch 5, dadurch gekennzeichnet, daß das Sterol Cholesterol ist.

7. Mittel nach Anspruch 5, dadurch gekennzeichnet, daß der anionische Stabilisator ausgewählt ist unter Mononatrium- oder Dinatriumacylglutamat, wobei der Acylrest ein C₁₄ - C₂₂-Rest ist, und Phosphorsäureestern von C₁₂-C₂₂- Fettalkoholen.

8. Mittel nach einem der Ansprüche 1 bis 7, dadurch gekennzeichnet, daß die Lipidphase 0,1 bis 8 Gew.-% bezogen auf das Gesamtgewicht des Mittels ausmacht.

9. Mittel nach einem der Ansprüche 1 bis 8, dadurch gekennzeichnet, daß die amphiphilen Lipide 0,1 bis 6 Gew.-% bezogen auf das Gesamtgewicht des Mittels ausmachen.

10. Mittel nach einem Ansprüche 1 bis 9, dadurch gekennzeichnet, daß der stabilisierende Zusatz weniger als 8 Gew.-% bezogen auf das Gesamtgewicht des Mittels ausmacht.

11. Mittel nach einem der Ansprüche 1 bis 10, dadurch gekennzeichnet, daß das ätherische Öl ausgewählt ist unter Eukalyptus-, Lavandin-, Lavendel-, Vetiver-, Litsea Cubeba-, Zitronen-, Sandelholzöl, Öl aus rotem oder weißem Thymian , Rosmarin-, Kamillen-, Pfefferkraut-, Muskatnuß-, Zimt-, Ysop-, Kümmel-, Orangen-, Gewürznelken-, Minz-, Rosen- und Persiliensamenöl.

12. Mittel nach einem der Ansprüche 1 bis 11, dadurch gekennzeichnet, daß die eingeschlossene wäßrige Phase und/oder die Lipidphase der Vesikel kosmetische und/oder pharmazeutische Wirkstoffe enthält.

13. Mittel nach einem der Ansprüche 1 bis 12, dadurch gekennzeichnet, daß die wäßrige Dispersionsphase wenigstens einen Zusatz enthält, ausgewählt unter Verdickungsmittel, Konservierungsmittel, Parfums, acidifizierenden Mitteln, alkalinisierenden Mitteln, stabilisierenden Mitteln, Farbstoffen und Sonnenfiltern.

14. Mittel nach einem der Anspruche 1 bis 13, dadurch gekennzeichnet, daß die Vesikel eine mittlere Größe im Bereich von 10 bis 1000 nm aufweisen und daß die ätherischen Öltröpfchen eine mittlere Größe im Bereich von 100 bis 10000 nm besitzen.

15. Verwendung eines Mittels nach einem der Ansprüche 1 bis 14 in Form einer Spülung, wobei der Gehalt des Mittels an ätherischem Öl (ätherischen Ölen) im Bereich von 3 bis 20 Gew.-% bezogen auf das Gesamtgewicht des Mittels beträgt.

16. Verwendung eines Mittels nach einem der Ansprüche 1 bis 14 in Form eines anderen Produkts als einer Spülung, wobei der Gehalt des Mittels an ätherischem Öl (ätherischen Ölen) im Bereich von 0,2 bis 3 Gew.-% bezogen auf das Gesamtgewicht des Mittels beträgt.
